# EUROPEAN PATENT SPECIFICATION

(11) **EP 3 549 598 B1**
(45) Date of publication and mention of the grant of the patent: **26.02.2025**
(21) Application number: 17876571.5
(22) Date of filing: 28.11.2017
(51) Int. Cl.: A61K 38/30, A61K 35/15, A61K 45/06, A61P 37/04, A61P 37/02, A61P 1/00, A61P 25/28, A61P 1/16, A61P 19/02, A61P 5/50, A61P 3/10, C12N 5/0786

(54) **PHARMACEUTICAL COMPOSITION CONTAINING INSULIN-LIKE GROWTH FACTOR-2 AND USE THEREOF**
PHARMAZEUTISCHE ZUSAMMENSETZUNG MIT INSULINÄHNLICHEM WACHSTUMSFAKTOR-2 UND DEREN VERWENDUNG
COMPOSITION PHARMACEUTIQUE CONTENANT UN FACTEUR DE CROISSANCE 2 DE TYPE INSULINE ET SON UTILISATION

(30) Priority: 29.11.2016 CN 201611073977
(43) Date of publication of application: 09.10.2019
(73) Proprietor: Shanghai Institute of Nutrition and Health, Chinese Academy of Sciences, Shanghai 200031 (CN)
(72) Inventor: SHI, Yufang, Shanghai 200031 (CN); WANG, Ying, Shanghai 200031 (CN); DU, Liming, Shanghai 200031 (CN); LIN, Liangyu, Shanghai 200031 (CN)
(74) Representative: dompatent von Kreisler Selting Werner - Partnerschaft von Patent- und Rechtsanwälten mbB
(86) International application number: PCT/CN2017/113341
(87) International publication number: WO 2018/099363

(56) References cited:
- WO-A1-93/25227
- WO-A2-2004/019965
- CN-A- 1 469 752
- CN-A- 105 007 942
- US-A1- 2003 027 755
- US-A1- 2014 255 413
- GUI YANG ET AL: "Insulin-like growth factor 2 enhances regulatory T-cell functions and suppresses food allergy in an experimental model", JOURNAL OF ALLERGY AND CLINICAL IMMUNOLOGY, vol. 133, no. 6, 1 April 2014 (2014-04-01), AMSTERDAM, NL, pages 1702 - 1708, XP055490485, ISSN: 0091-6749, DOI: 10.1016/j.jaci.2014.02.019
- UCHIMURA, T. ET AL.: "Insulin-like growth factor II (IGF-II) Inhibits IL -1 0 -Induced Cartilage Matrix Loss and Promotes Cartilage Integrity in Experimental Osteoarthritis", JOURNAL OF CELLULAR BIOCHEMISTRY, vol. 116, no. 12, May 2015 (2015-05-01), pages 2858 - 2869, XP055490477
- CHEN, J. ET AL.: "Regulation of PD-LI: a novel role of pro-survival signalling in cancer", ANNALS OF ONCOLOGY, vol. 27, no. 3, 17 December 2015 (2015-12-17), pages 409 - 416, XP055362125
- HAMAMURA, K. ET AL.: "IGF2-driven PI3 kinase and TGF 0 signaling pathways in chondrogenesis", CELL BIOLOGY INTERNATIONAL, vol. 32, no. 10, 31 December 2008 (2008-12-31), pages 1238 - 1246, XP025479905
- YANG, G. ET AL.: "Insulin-like growth factor 2 enhances regulatory T-cell functions and suppresses food allergy in an experimental model.", J. ALLERGY. CLIN. IMMUNOL, vol. 133, no. 6, 1 April 2014 (2014-04-01), pages 1702 - 1708, XP055490485
- YANG, LIJUAN ET AL.: "Progress in relationship between Thl7 and Th1/Treg cell", IMMUNOLOGICAL JOURNAL, vol. 26, no. 4, 30 April 2010 (2010-04-30), pages 353 - 355, XP009515533, DOI: 10.13431/j.cnki.immunol.j.20100083

## Description

### Field of the invention

This invention relates to the field of biological medicine, and in particular to a pharmaceutical composition containing insulin-like growth factor-2 and use thereof.

### Background

Macrophages are specialized immune cells distributed in all parts of the body. They remove the senescence cells, certain cells that cannot be recognized as their own, cell debris and so on through phagocytosis. As the professional antigen presenting cells, macrophages initiate the immune responses of unsensitized T cells and trigger the inflammatory response. However, inflammatory cytokines expressed by macrophages such as TNFα and IL-1 also play important roles in the pathogenesis of many autoimmune diseases. Removal of pathogenic macrophages has been shown to significantly inhibit autoimmune encephalomyelitis and inflammatory bowel diseases. In addition to their pro-inflammatory properties, macrophages can be regulated by different circumstances factors into cells with immunosuppressive effect, such as macrophages marked by expressing IL-10, Arginase I, etc, as well as macrophages with immunosuppressive function which are formed by different immune stimuli in the evolution process of monocytes to macrophages, thereby directly or indirectly regulating the body's immune response. It has been reported that spermidine treated macrophages are able to powerfully treat autoimmune diseases, indicating that macrophages with immunosuppressive effect have significant therapeutic effects in autoimmune diseases.

Currently, there is limited understanding on the application of macrophages to treat autoimmune diseases. New therapeutic strategies employing the plasticity of macrophages for autoimmune diseases are urgently need in the art.

WO 2004/019965 A2 discloses a thymus-based tolerogenic approaches for type 1 diabetes.

US 2003/0027755 A1 discloses methods and compositions for the protection of white matter of the central nervous system including the use IGF 1.

Gui Yang et al. in Journal of Allergy and Clinical Immunology 133 (2014), 1702 - 1708 discloses that insulin-like growth factor 2 enhances regulatory T-cell functions and suppresses food allergy in an experimental model.

WO 93/25227 A1 discloses the use of growth factor IGF-II.

US 2014/0255413 A1 discloses an insulin-like growth factor receptor antagonist for use in the treatment of neoplasia.

### Summary of the invention

The present invention provides Macrophage treated with insulin-like growth factor-2 (IGF-2) for use in the treatment of an autoimmune disease.

In a preferred embodiment, the IGF-2 includes full length IGF-2 and an active fragment of IGF-2.

In another preferred embodiment, the active fragment of IGF-2 is an active fragment having amino acids from position 25 to position 91 of IGF-2.

In another preferred embodiment, the active fragment of IGF-2 is the amino acid sequence of position 25 to position 91 of IGF-2.

In another preferred embodiment, the IGF-2 is from human and non-human mammals.

In another preferred embodiment, the amino acid sequence of the IGF-2 is shown in SEQ ID NO.:1.

In another preferred embodiment, the autoimmune disease is selected from the group consisting of multiple sclerosis, inflammatory bowel disease, autoimmune encephalomyelitis, autoimmune hepatitis, systemic lupus erythematosus, rheumatoid arthritis, insulin resistance, diabetes, liver cirrhosis or a combination thereof.

In another preferred embodiment, the insulin resistance or the diabetes is obesity-induced insulin resistance or obesity-induced diabetes.

The third aspect of the invention provides a pharmaceutical composition comprising (a) IGF-2 or the active fragment thereof (such as active fragment having amino acids from position 25 to position 91), (b), macrophage scavenger, (c) optionally, PD-L1 promoter, and (d) a pharmaceutically acceptable carrier as defined in claim 7.

In another preferred embodiment, the macrophage scavenger is an agent for removing macrophage or inhibiting macrophage migration.

In another preferred embodiment, the formulation of the pharmaceutical composition is an injection formulation, sustained release formulation or topical formulation.

In another preferred embodiment, the pharmaceutical composition is used for one or more of the purposes selected from the group consisting of:
(a) promoting PD-L1 expression in macrophage;
(b) inhibiting IL-1β expression in macrophage;
(c) promoting differentiation of regulatory T cell;
(d) inhibiting differentiation of Th1 cell and/or Th17 cell; and
(e) treating autoimmune disease.

The pharmaceutical composition comprises macrophage scavenger which depletes macrophage or suppresses macrophage migration.

The macrophage scavenger is selected from the group consisting of: CCR2 inhibitor (to inhibit macrophage migration), clodronate disodium liposome (to remove macrophage) and a combination thereof.

In another preferred embodiment, if the pharmaceutical composition comprises component (a), component (b) and component (c), the mass ratio of component (a): component (b): component (c) is (1-100): (1-100): (1-100).

In another preferred embodiment, if the pharmaceutical composition comprises component (a) and component (b), the mass ratio of component (a): component (b) is (1-100): (1-100).

In another preferred embodiment, if the pharmaceutical composition comprises component (a) and component (c), the mass ratio of component (a): component (c) is (1-100): (1-100).

In another preferred embodiment, the ratio (mg: mg) of IGF-2 active fragment having amino acids from position 25 to position 91 to macrophage scavenger ranges from 1:100 to 100:1, preferably, from 1:20 to 20:1.

In another preferred embodiment, the total content of IGF-2 active fragment having amino acids from position 25 to position 91 and macrophage scavenger is 1 to 99 wt% of the pharmaceutical composition, preferably, 5 to 90wt% of the total pharmaceutical composition.

The invention provides a macrophage pre-treated with IGF-2.

In another preferred embodiment, in the macrophage, the PD-L1 expression is up-regulated and/or (ii) the IL-1β expression is down-regulated.

In another preferred embodiment, the up-regulation referred to that the ratio of PD-L1 expression quantity in treated macrophage (M1) to PD-L1 expression quantity in untreated macrophage (M0) M1/M0 is ≥1.5, preferably, M1/M0≥2.0, more preferably, M1/M0≥3.0. In another preferred embodiment, the down-regulation referred to that the ratio of IL-1β expression quantity in untreated macrophage (N0) to IL-1β expression quantity in treated macrophage (N1) N0/N1 is ≥1.5, preferably, N0/N1≥2.0, more preferably, N0/N1≥3.0.

In another preferred embodiment, the treatment includes exposing macrophage to IGF-2 or IGF-2 active fragment for a period of time (such as 0.1-24 hours).

A further aspect of the invention provides a pharmaceutical composition for regulating T cell differentiation, which comprises IGF-2 treated macrophage and a pharmaceutically acceptable carrier, wherein the pharmaceutical composition is used for treating autoimmune disease.

A further aspect of the invention provides an in-vitro, non-therapeutic method for (i) promoting PD-L1 expression and/or (ii) inhibiting IL-1β expression in macrophage, which comprises the following steps:
(a) culturing macrophage in the presence of IGF-2 to (i) enhance PD-L1 expression and/or (ii) inhibit IL-1β expression.

In another preferred embodiment, the concentration of IGF-2 is 1 ng/ml-100 µg/ml, preferably, 3 ng/ml-1 µg/ml, and more preferably, 5 ng/ml-50 ng/ml.

Preferred is a polypeptide with a sequence of position 25 to position 91 of SEQ ID NO.: 1.

The invention provides a use of the pharmaceutical composition for treating an autoimmune disease.

It should be understood that in the present invention, the technical features specifically described above and below (such as the Examples) can be combined with each other, thereby constituting a new or preferred technical solution, which needs not be described one by one, due to space limitations.

### Brief description of drawings

Figure 1 shows that insulin-like growth factor 2 (IGF-2) is effective in treating EAE.
Figure 1a shows that IGF-2 can effectively alleviate the progress and degree of EAE.
Figure 1b shows that IGF-2 can effectively inhibit mononuclear cell infiltration in the central nervous system.
Figure 1c shows that IGF-2 can effectively inhibit MOG specific T cell proliferation in EAE mice.
Figure 1d, 1e, 1f, and 1g show that IGF-2 can effectively inhibit the cell proportion of Th1 and Th17 in EAE mice and promote the proportion of Treg.
Figure 1h shows that IGF-2 does not directly effect the differentiation of Th1/Th17/Treg.
Figure 1i and 1j show that IGF-2 promotes PD-L1 expression and inhibits IL-1β expression in the CD11b+F4/80+ macrophage in the spinal cord of EAE mice.
Figure 2 shows that insulin-like growth factor-2 (IGF-2) can induce anti-inflammatory macrophages.
Figure 2a shows that IGF-2 does not affect the number of macrophages in the spinal cord of EAE mice.
Figure 2b shows that IGF-2 does not affect the number of macrophages in the spleen of EAE mice.
Figure 2c shows that IGF-2 inhibits the expression level of IL-1β in CDllb+ F4/80+ macrophages in the spleen of EAE mice.
Figure 2d shows that IGF-2 promotes the expression level of PD-L1 in CDllb+ F4/80+ macrophages in the spinal cord of EAE mice.
Figure 2e shows that IGF-2 does not affect the expression levels of MHC-I, MHC-II, CD80, and CD86 in thioglycollate medium-induced peritoneal macrophages.
Figure 2f shows that IGF-2 does not affect the expression levels of TNF-α, IL-6 and TGF-β in peritoneal macrophages under lipopolysaccharide (LPS) stimulation and thioglycollate medium-induction.
Figure 2g shows that IGF-2 inhibits the mRNA levels of inducible nitric oxide synthase (iNOS) and the production of nitrite regulated by the enzyme, in the peritoneal macrophages under lipopolysaccharide (LPS) stimulation and thioglycollate medium-induction.
Figure 3 shows that insulin-like growth factor-2 (IGF-2) treated macrophages are effective in treating EAE.
Figure 3a shows that after IGF-2 pretreatment, under 100 ng/ml LPS stimulation, the ability of peritoneal macrophages induced by thioglycollate medium to express messenger RNA and precursor proteins of IL-1β is significantly decreased.
Figure 3b shows that after IGF-2 pretreatment, under 100 ng/ml LPS stimulation, the IL-1β protein level secreted by peritoneal macrophages induced by thioglycollate medium is significantly decreased.
Figure 3c shows that after IGF-2 pretreatment, under 100ng/ml LPS stimulation, the PD-L1 protein level expressed by peritoneal macrophages induced by thioglycollate medium is significantly increased.
Figure 3d and 3e show that IGF-2 treated macrophages are able to promote Treg differentiation depend on PD-L1.
Figure 3f shows that IGF-2 treated macrophages significantly inhibits EAE.
Figure 3g shows that IGF-2 treated macrophages can effectively promote the proportion of Tregs in EAE mice.
Figure 4 shows that bone marrow derived macrophages treated with insulin-like growth factor-2 (IGF-2) to promote the differentiation ability of regulatory T cell.
Figure 4a shows that bone marrow derived macrophages treated with IGF-2 express significant higher level of PD-L1 under LPS stimulation.
Figure 4b and 4c show that bone marrow derived macrophages treated with IGF-2 promote Treg through PD-L1.
Figure 4d shows no change in the expression levels of MHC-I, MHC-II, CD80, and CD86 in bone marrow derived macrophages treated with IGF-2.
Figure 5 shows that bone marrow derived macrophages treated with insulin-like growth factor-2 (IGF-2) are effectively in alleviating experimental autoimmune encephalomyelitis (EAE) and inflammatory bowel disease (IBD).
Figure 5a shows that bone marrow derived macrophages treated with IGF-2 are effective in treating EAE.
Figure 5b and 5c show that bone marrow derived macrophages treated with IGF-2 effectively increase the proportion of Treg in EAE mice.
Figure 5d shows that bone marrow derived macrophages treated with IGF-2 relieved weight loss in IBD mice. Macrophages treated with IGF-2 shows the ability to effectively treat IBD.
Figure 5e shows that bone marrow derived macrophages treated with IGF-2 can effectively prolong the survival time of mice with IBD.
Figure 5f and 5g show that bone marrow derived macrophages treated with IGF-2 effectively increase the proportion of Treg cells in spleen in the IBD mice.
Figure 6 shows that insulin-like factor-2 (IGF-2) affects the anti-inflammatory prosperities of macrophages through regulating their glycometabolism.
Figure 6a shows that glucose consumption was significantly reduced in macrophages after IGF-2 treated.
Figure 6b shows that lactic acid accumulation was significantly reduced in macrophages after IGF-2 treated.
Figure 6c shows that NAD+/NADH ratio was significantly reduced in macrophages after IGF-2 treated.
Figure 6d shows that the ability of oxidative phosphorylation and oxygen consumption was significantly increased in macrophages after IGF-2 treated.
Figure 6e shows that extracellular acidification was significantly reduced in macrophages after IGF-2 treated.
Figure 6f shows that macrophages preferred to conduct oxidative phosphorylation after IGF-2 treated.
Figure 6g shows that oligomycin could inhibit the improvement of PD-L 1 expression in peritoneal macrophages treated with IGF-2 and induced by thioglycollate medium.
Figure 6h shows that oligomycin inhibits oxidative phosphorylation, and demonstrates that macrophages treated with IGF-2 have high expression of PD-L1 depended on aerobic respiration and the ability to promote the production of Treg.
Figure 7 shows that insulin-like growth facor-2 (IGF-2) and IGF-2 treated macrophages are effective in treating experimental bowel disease.
Figure 7a shows that IGF-2 protects IBD mice from body weight loss.
Figure 7b shows that IBD mice received IGF-2 treatment have significant longer colon length.
Figure 7c shows that numbers of mononuclear cells infiltration significantly decrease in colon after IGF-2 treatment.
Figure 7d and 7e show that the proportion of Treg cells in the lamina propria of colon significantly increase in the IBD mice after IGF-2 treatment.
Figure 7f shows that the expression of PD-L1 significantly increases in CD11b+F4/80+ macrophages in the lamina propria of colon of the IBD mice after IGF-2 treatment.
Figure 7g shows that the expression level of IL-1β significantly decrease in CD11b+F4/80+ macrophages in the lamina propria of colon of the IBD mice after IGF-2 treatment.
Figure 7h shows that IGF-2 treated macrophage relieves the body weight loss of experimental bowel disease mice.
Figure 7i shows that IGF-2 treated macrophage effectively prolongs the survival time of the IBD mice.
Figure 7j shows that the proportion of Treg cells in spleen significantly increase in the IBD mice injected with IGF-2 treated macrophage.
Figure 8 shows that insulin-like growth factor-2 (IGF-2) regulates immune response in the spleen and peritoneal lymph nodes of inflammatory bowel disease (IBD) mice.
Figure 8a and 8b show that IGF-2 significantly up regulates the proportion of Tregs in the peritoneal lymph nodes of mice with IBD.
Figure 8c shows that IGF-2 does not affect the proportion of spleen macrophages in mice with IBD.
Figure 8d shows that IGF-2 upregulates the proportion of CD11b+F4/80+ macrophages in the colon lamina propria of mice with IBD.
Figure 8e shows that IGF-2 significantly downregulates the expression level of IL-1β in spleen macrophages in mice with IBD.
Figure 8f shows that IGF-2 significantly up regulates the expression level of PD-L1 in spleen macrophages in mice with IBD.
Figure 9 shows that peritoneal macrophages treated with insulin-like growth factor-2 (IGF-2) and induced by thioglycollate medium can upregulate Tregs in lamina propria of colon and peritoneal lymph nodes of IBD mice.
Figure 9a shows that IGF-2 treated macrophages can significantly increase the proportion of Tregs in peritoneal lymph nodes of IBD mice.
Figure 9b shows that IGF-2 pre-treated macrophages can significantly increase the proportion of Tregs in lamina propria of colon of IBD mice.
Figure 10 shows that separate injection of insulin-like growth factor-2 (IGF-2) and clodronate liposome can significantly inhibit the progress of EAE and play an effective therapeutic role when compared with that of control mice group. Importantly, combined injection of IGF-2 and clodronate liposome has significantly better therapeutic effects on EAE than other groups.
Figure 11 shows the tertiary structure of insulin-like growth factor-2 (IGF-2).
Figure 12 shows the key binding sites of insulin-like growth factor-2 (IGF-2) to the receptor.
Figure 13A shows the results of glucose tolerance test.
Figure 13B shows the results of insulin tolerance test.

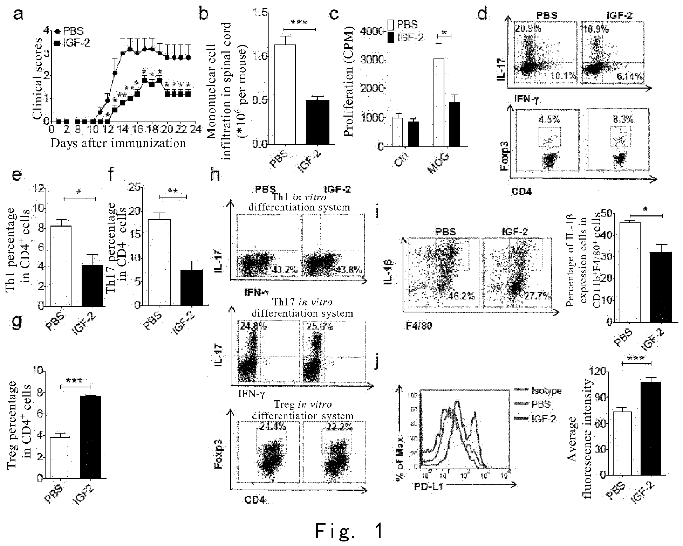

IGF-2 applied in the reference and description of figures 1-13 is active fragment of IGF-2 containing amino acids from position 25 to position 91. (IGF₂₅₋₉₁)

### Detailed description of the invention

The invention is defined in the claims.

This invention provides the use of IGF-2 and its active fragment for preparing a pharmaceutical composition. The pharmaceutical composition can be used for (i) enhancing PD-L1 expression and/or (ii) inhibiting IL-1β expression in macrophages. The invention also provides a pharmaceutical composition containing insulin-like growth factor-2 as an active ingredient.

Specifically, in the invention, it is found that both insulin-like growth factor-2 (IGF-2) and the active fragment having amino acids from position 25 to position 91 of IGF-2 (IGF₂₅₋₉₁) show significant therapeutic effects in the treatment of animal models of multiple sclerosis and colitis (experimental autoimmune encephalomyelitis and DSS-induced inflammatory bowel diseases), and can significantly inhibit the progression of the disease and the tissue damage and inflammatory infiltration in corresponding site. More importantly, IGF₂₅₋₉₁ indirectly upregulates regulatory T cells (Treg) by inducing macrophages with low IL-1 expression and high PD-L1 expression, thereby promoting the recovery of autoimmune diseases. IGF₂₅₋₉₁ mainly promotes the reprogramming of macrophages into macrophages with immunosuppressive function by affecting the aerobic glycolysis and oxidative phosphorylation pathways of macrophages, and such macrophages treated with IGF₂₅₋₉₁ have a very significant effect on the treatment of autoimmune diseases. Based on the therapeutic effect of IGF₂₅₋₉₁ on autoimmune diseases, the present invention clarifies the regulatory mechanism of IGF₂₅₋₉₁ on macrophages, and it is found that IGF₂₅₋₉₁ induces the application potential of macrophages with immunosuppressive function in the treatment of autoimmune diseases, thus forming a new technique for macrophage immunotherapy.

### Insulin-like growth factor-2

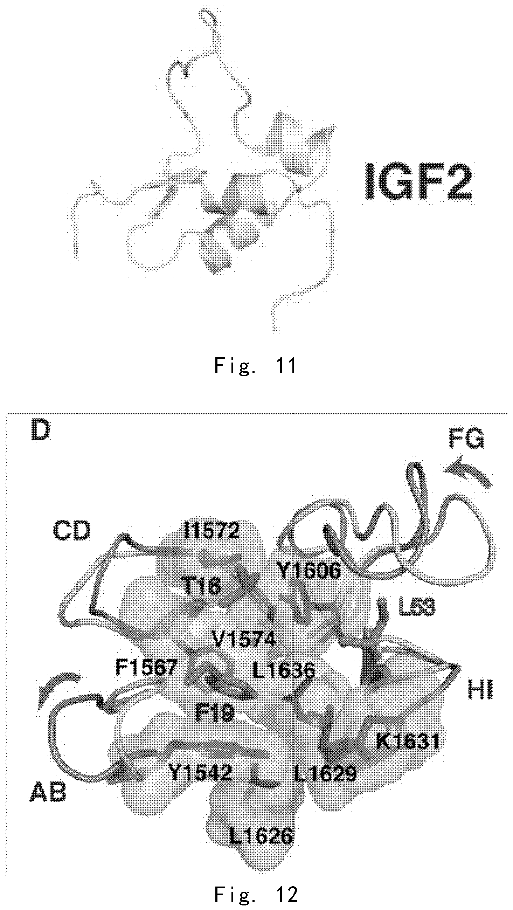

Insulin-like growth factor-2 (IGF-2) is a growth factor mainly synthesized in the liver and is abundantly present in the blood. IGF-2 has anti-apoptosis, growth regulation, insulin-like and mitotic functions. IGF-2 precursor protein , which is translated from mRNA, consists of 180 amino acids, as shown in the sequence SEQ ID NO.: 1 (mgipmgksmlvlltflafascciaayrpset lcggelvdtlqfvcgdrgfyfsrpasrvsrrsrgiveeccfrscdlalletycatpakserdvstpptvlpdnfprypvgkffqydtwkqst qrlrrglpallrarrghvlakeleafreakrhrplialptqdpahggappemasnrk); The active form produced after modification after translation consists of 67 amino acids, as shown in the sequence SEQ ID NO.: 2 (ayrpsetlcggelvdtlqfvcgdrgfyfsrpasrvsrrsrgiveeccfrscdlalletycatpakse). The tertiary structure of IGF-2 is composed of three α-helixes and two β-sheets as shown in Figure 11, wherein amino acids 11G-21C forms a helix, 25G-27Y forms the first sheet, the second helix is 42I-49R, the third helix is 53L-58T and the second sheet is 59Y-61A. It is predicted that T16 and F19 on the first helix and L63 on the third helix are sites where IGF-2 binds to receptor of IGF-2 as shown in Figure 12.

At present, it is generally believed that IGF-2 has an important role in embryo development and can promote embryo development and organ formation. It has also been reported to be linked to memory and reproduction, and studies in genetically deficient mice have shown that lack of insulin-like growth factor-2 signaling can lead to poor brain development.

The present invention has found that administration of IGF-2 or active fragment having the amino acids sequence from position 25 to position 91 of IGF-2 (IGF₂₅₋₉₁) alone can effectively treat experimental autoimmune encephalomyelitis or inflammatory bowel disease, which are two autoimmune diseases. In the lesion site, such as the spinal cord or colon of the diseased mice, the proportion of regulatory T cells in the IGF₂₅₋₉₁ treatment group was significantly increased, and the proportions of TH1 and TH17 cells were significantly decreased.

However, when the *in vitro* T cell differentiation system was used to verify the effect of IGF₂₅₋₉₁ on T cell differentiation, it was found that IGF₂₅₋₉₁ could not directly affect the efficiency of T cell differentiation into Th1, Th17 and Treg. These studies suggest that IGF₂₅₋₉₁ has an indirect effect on the proportion change of T cell subsets. Significant changes in the macrophage phenotype were also observed when insulin-like growth factor-2 was used to treat autoimmune diseases. It was found in the study that the expression of IL-1β in macrophages at the injury site of mice treated with IGF₂₅₋₉₁ was significantly decreased, while the expression of PD-L1 was significantly increased, suggesting that IGF₂₅₋₉₁ played a role in inhibiting autoimmune diseases through promoting the proportion of macrophages with anti-inflammatory effects.

It is found in the study that IGF₂₅₋₉₁ does not directly induce the expression of anti-inflammatory genes in macrophages. However, the bone marrow or enterocoelia derived mature macrophages treated with IGF₂₅₋₉₁ exhibit significantly decrease of IL-1β expression and significantly increase of PD-L1 expression in response to lipopolysaccharide stimulation. Macrophages play an important role in the occurrence and development of autoimmune diseases. A series of studies have shown that macrophages can exert strong proinflammatory ability under the stimulation of inflammatory factors such as interferon and lipopolysaccharide, which aggravate the process of autoimmune diseases. Studies have shown that insulin-like growth factor-2 alters the response of macrophages to inflammatory factors, and macrophages overexpress the anti-inflammatory molecule PD-L1 under the condition of proinflammatory stimulation. Therefore, the treatment of autoimmune diseases by IGF₂₅₋₉₁ is likely to be achieved by reprogramming macrophages.

In order to clarify the role of macrophages treated with IGF₂₅₋₉₁ in regulating Treg, macrophages induced by IGF₂₅₋₉₁ were co-cultured with T cells after LPS pre-stimulation. It was found that macrophages treated with IGF₂₅₋₉₁ could significantly promote the differentiation of Treg, and the promotion depended on the high expression of PD-L1. Further, the studies showed that macrophages treated with IGF₂₅₋₉₁ could also be directly used to treat autoreactive encephalomyelitis and inflammatory bowel disease. During the treatment process, the infusion of these macrophages also promoted the increase of Treg proportion in mice.

In order to further explain how IGF₂₅₋₉₁ affects the expression of PD-L1 in macrophages, a detailed comparison was made between macrophages treated with IGF₂₅₋₉₁ and control cells. It was found that macrophages treated with IGF₂₅₋₉₁ can significantly change the status of glucose metabolism pathway of macrophages, making them more apt to oxidative phosphorylation metabolic pathway. When blocking macrophages mitochondrial oxidative phosphorylation, the high expression of PD-L1 and the ability to promote Treg differentiation induced by insulin-like growth factor - 2 in macrophages also disappeared. These studies illustrate that IGF₂₅₋₉₁ endows macrophages with the ability of promoting Treg generation and treating autoimmune diseases by changing the metabolism tendencies of macrophages.

In conclusion, IGF₂₅₋₉₁ plays a therapeutic role in multiple sclerosis and inflammatory bowel disease by inducing immunosuppressive macrophages and up-regulating regulatory T cells. Macrophages treated with IGF₂₅₋₉₁ may play a therapeutic role in autoimmune diseases. In addition, the targeted regulation of aerobic glycolysis and oxidative phosphorylation in macrophages can induce the production of immunosuppressant macrophages and play a role in the treatment of various autoimmune diseases.

### Pharmaceutical composition and instructions

This invention provides a pharmaceutical composition for (i) promoting PD-L1 expression and/or (ii) inhibiting IL-1β expression in macrophages. It contains a safe and effective dose of IGF-2 (or its active fragments) or contains IGF-2 treated macrophages.

The pharmaceutical composition of the invention can be used for (i) promoting PD-L1 expression and/or (ii) inhibiting IL-1β expression in macrophages. The pharmaceutical composition can be used together with other autoimmune diseases therapies.

The pharmaceutical composition of the invention also contains macrophage scavenger which is an agent for removing macrophages or inhibiting macrophage migration. Experiments showed that the combination therapy of IGF-2₂₅₋₉₁ and macrophage scavenger played a more optimized role in the treatment of inflammatory diseases by reprogramming macrophages with immunosuppressive function.

The invention also disclose a medicine kit comprising:
(i) a first container which contains an active ingredient (a) IGF-2 or a medicine containing the active ingredient (a);
(ii) a second container which contains an active ingredients (b) macrophage scavenger or a medicine containing the active ingredient (b), wherein the macrophage scavenger is agent for removing macrophage or inhibiting macrophage migration, and
(iii) an specification describing a combined administration of the active ingredient (a) and the active ingredient (b) for treating autoimmune diseases.

The pharmaceutical composition of the invention contains a safe and effective dose of IGF-2 and a pharmaceutically acceptable carrier. The carrier includes (not limited to): saline, buffer solution, glucose, water, glycerin, alcohol, powder and the combination thereof. The formulation of the medicine should be compatible with the method of administration.

The pharmaceutical composition of the invention can be prepared in the form of an injection, for example, by a conventional method using saline or an aqueous solution containing glucose and other adjuvants. Pharmaceutical compositions such as tablets and capsules may be prepared by conventional methods. Pharmaceutical composition such as injection, solution, tablet and capsule should be manufactured under sterile conditions. The pharmaceutical composition of the invention can also be made into powder for atomizing inhalation. The dose of the active ingredient is an effective dose, such as about 1 µg/kg-50 mg/kg, about 5 µg/kg-10 mg/kg, about 10 µg/kg-5 mg/kg of body weight per day. The pharmaceutical composition can be administered together with other therapies.

The pharmaceutical composition of the invention can be administered to a subject (such as human and non-human mammal) in routine manner. The representative way for administration includes (but not limit to): oral administration, injection and aerosol inhalation.

The pharmaceutical composition should be administered to a mammal in a safe and effective dose. The safe and effective dose is usually at least 10 µg/kg of body weight and no more than 50 mg/kg of body weight under most conditions. The preferred dose should range from 10 µg/kg to 20 mg/kg of body weight. The exact dose should be determined according to the administration route and the patient health conditions which are all within the expertise of a skilled physician.

### The main advantages of the present invention includes:

(a) IGF-2 and the active fragment thereof (from position 25 to position 91) can (i) promote PD-L1 expression and/or (ii) inhibit IL-1β expression in macrophages.
(b) Macrophages treated with IGF-2 or the active fragment thereof can promote Treg differentiation and inhibit Th1 cell and/or Th17 cell differentiation so as to treat autoimmune diseases.
(c) Combination therapy of IGF-2 or active fragment thereof with macrophage scavenger can be used for treating autoimmune diseases.

The present invention will be further illustrated below with reference to the specific examples. It should be understood that these examples are only to illustrate the invention but not to limit the scope of the invention. For the experimental methods in the following examples the specific conditions of which are not specifically indicated, they are performed under routine conditions, or as instructed by the manufacturers, unless otherwise specified. Unless indicated otherwise, parts and percentage are weight parts and weight percentage.

### Materials and Methods

### Establishment and treatment of experimental autoreactive encephalomyelitis EAE model

### (1) Preparation before experiment

Preparation of complete freund's adjuvant: heat inactivated mycobacterium tuberculosis (purchased from sigma) was added into incomplete freund's adjuvant until the final concentration reached 5mg/ml, and the solution were thoroughly mixed before use.

Emulsification of antigen: Two glass syringes were connected through a three-way tube, an equal volume of antigen solution (300 µg MOG in 100 µL PBS) and 100 µL of complete Freund's adjuvant were added into the syringes, and the bubbles in the syringes were removed and the syringes were pushed back and forth to obtain an emulsion. After about 500 pushes, the push resistance would gradually increase indicating the ingredients were fully mixed into an emulsified state.

Preparation of pertussis toxin (PT) solution: pertussis toxin was dissolve in PBS at a working concentration of 1 ng/µL and 200 µL per mouse was injected through the tail vein.

### (2) Establishment and treatment of EAE model

On day 0, 200 µL of the emulsified antigen was subcutaneously injected into the back of C57BL/6 mice (purchased from the Slack Shanghai Laboratory Animal Center of the Chinese Academy of Sciences) for antigen immunization, and 100 µL was injected into each side of the chest corresponding to the back. Meanwhile, 200 µL/mouse of PT was injected through the tail vein.

On day 2, 200 µL/mouse of PT was injected again through the tail vein.

From the ninth day, insulin-like growth factor 2 was used for treatment via intraperitoneal injection at a dose of 5 ng/mouse everyday. The saline treated group served as a positive control.

When macrophages were used for treatment, 5×10⁶ macrophages were injected intraperitoneally on Day 9, 11, and 13, respectively, and clinical scores were recorded daily.

### (3) Clinical scoring of EAE

0: clinically normal; 0.5: tail drooping at the tip; 1: whole tail paralysis; 2: hind legs weakness; 3: one hind limb paralysis; 4. both hind limbs paralysis; 5: died.

### 2. Extraction of lymphocytes from spinal cord

The spinal cord was removed from EAE mice and washed with PBS. The spinal cord was mechanically ground on a 70 µm pore size cell screen to obtain a single cell suspension. The obtained cell suspension was centrifuged, and the cells were resuspended in 30% percoll, and an equal volume of 70% percoll solution was gently added to the bottom. Centrifugation was carried out at 2000 rpm for 20 minutes at room temperature under zero acceleration. After centrifugation, cells were aspirated from the liquid junction layer of high-low density and washed twice with PBS to obtain lymphocytes.

### 3. In vitro proliferation experiment of splenocytes

After the EAE mice were sacrificed, the spleen of the mice was removed and a single cell suspension was obtained. The splenocytes were plated in a U-bottom 96-well plate at a number of 3-5×10⁵ per well, and MOG was added to 20 ug/ml.

After incubation at 37 ° C for 72 hours in an incubator, H3 labeled thymidine was added. After 6 hours, the well plate was freeze-thawed repeatedly and then vacuum-adsorbed onto a special filter. The scintillation solution was added and then detected on the machine.

### 4. Establishment and treatment of inflammatory bowel disease IBD model

Dextran sodium sulfate (DSS) was formulated into a solution at a mass to volume ratio of 2.5: 100, filtered with a 22 µm strainer to ensure sterility, and sealed with a parafilm.

8-10 weeks aged C57/BL6 female mice (purchased from Slack Shanghai Laboratory Animal Center of the Chinese Academy of Sciences) were selected and used. Dextran sodium sulfate solution was used to replace the drinking water to induce the model. The mice weight and feces were observed every day. The dextran sulfate sodium solution was changed or added every other day.

From the day of model induction, insulin-like growth factor-2 was injected intraperitoneally at a dose of 50 ng/mouse everyday. The saline treated group served as a positive control.

When macrophages were used for treatment, 5×10⁷ macrophages were injected intraperitoneally on Day 1, 3, and 5, respectively, and the change of body weight was recorded daily.

### 5. Extraction of lymphocyte from colon tissue

The colon was removed from the IBD mice, the feces were removed and washed with PBS. Then the colon was cut into 2 cm sections and rinsed twice in HBSS (1 mM DTT and 5 mM EDTA) under conditions of 250 rpm for 20 min at 37 °C. The washed colon tissue was cut as much as possible with scissors and added into HBSS containing DNAase I, dispase and type VIII collegenase under the conditions of 250 rpm for 30 min at 37 °C. The obtained cell suspension was centrifuged, and the cells were resuspended in 40% percoll, and an equal volume of 80% percoll solution was gently added to the bottom. Centrifugation was carried out at 2000 rpm for 20 minutes at room temperature under zero acceleration. After centrifugation, cells were aspirated from the liquid junction layer of high-low density and washed twice with PBS to obtain lymphocytes.

### 6. Hippocampal test

Cells were implanted on the hippocampal test plate at a density of 1×10⁵ cells per well. XF culture was supplemented with 10mM glucose, 2 mM glutamine and 2 mM pyruvate to prepare a test medium. The cells were washed twice with the test medium before testing on the machine. Metabolic inhibitors were prepared using test medium: 1 µM oligomycin, 0.75 µM FCCP, 100 µM rotenone and 1 µM Mantimycin. The consumption rate of oxygen and the concentration of extracellular hydrogen ion at basic status and under drug stimulation conditions were measured. The instrument used was Extracellular flux analyzer.

### 7. Preparation and treatment of macrophages

The preparation process of bone marrow derived macrophages was as follows: mouse bone marrow was blown out with 1 ml syringe. After being full dispersed, the cells were centrifuged according to the condition of 400 g for 5 min. The supernatant was discarded, the cells were blown into single cell suspension, and the cells were laid on an uncoated culture dish in DMEM/F12 completely medium (containing 10% FBS, 20% L929 supernatant, 100 U/ml Penicillin, 100 U/ml Streptomycin, 2 mm L - Glutamine). At the fourth day of culture, 5ml solution was added, and the cells were further cultured for 6 days, and mature macrophages could be obtained. IGF-2 stimulation was added on Day 1, 3 and 5, respectively.

The preparation process of peritoneal derived macrophages was as follows: 2 ml of 4% thioglycollate solution was intraperitoneally injected into 8-12 weeks aged C57BL/6 mice, cytokines or PBS were intraperitoneally injected daily every day. On the third day, mice were sacrificed. Macrophages in abdominal cavity were blown out by using a 10 ml syringe with 10 ml volume of PBS. After centrifugation, the cells were scattered to form a single cell suspension, and were then inoculated in an uncoated cell dish or a 6-well plate, for following experiments.

### 8. Co-culture system of macrophages and naïve T Cells

Macrophages derived from mature bone marrow or peritoneal were stimulated with LPS for 24 hours. The stimulation solution was aspirated and cells were washed with PBS for three times to fully remove the residual LPS. Then, the macrophages (2.5×10⁵) and selected CD4⁺CD62L⁺ T cells (1×10⁶) were laid on a 96-well plate. After 72 hours of co-culture, the cells were blown out for flow cytometry staining and analysis.

### 9. Establish obesity modeling by high-fat diet induction

C57/BL6 mice (purchased from slake Shanghai experimental animal center of Chinese academy of sciences) were fed with a high-fat diet for 20 weeks, starting from the 5th week.

### 10. Glucose tolerance test

The mice were starved overnight, and 1g glucose/kg body weight was intraperitoneally injected. At different time points, a drop of tail venous blood of each mouse was siphoned into the blood glucose test paper, and blood glucose was measured with a blood glucose meter.

### 11. Insulin tolerance test

The mice were starved overnight for about 16 hours, and 0.75U insulin /kg body weight was intraperitoneally injected. At different time points, a drop of tail venous blood of each mouse was siphoned into the blood glucose test paper, and blood glucose was measured with a blood glucose meter, and accurate timing was conducted with a timer.

### Example 1

### IGF₂₅₋₉₁ can treat experimental autoimmune encephalomyelitis (EAE) depending on its regulation of gene expression in macrophages

When treated with IGF₂₅₋₉₁ in mice induced EAE, it was observed that IGF₂₅₋₉₁ effectively alleviated the pathogenesis and severity of EAE, which was manifested by decreased clinical score, decreased number of infiltrating mononuclear cells in the spinal cord, and decreased proliferation of MOG-specific T cells in EAE mice (Figures 1 a-c). Meanwhile, flow cytometry analysis showed that the use of IGF₂₅₋₉₁ could effectively reduce the proportions of Th1 and Th17 in the spinal cord of EAE mice and increase the proportion of Treg (Figures 1d-g). However, when the inventor added IGF₂₅₋₉₁ directly into an *in vitro* differentiation system of T cells, it was found that the differentiation efficiency of T cells into Th1, Th17 and Treg was not affected, suggesting that IGF₂₅₋₉₁ had an indirect effect on T cells (Figure 1h).

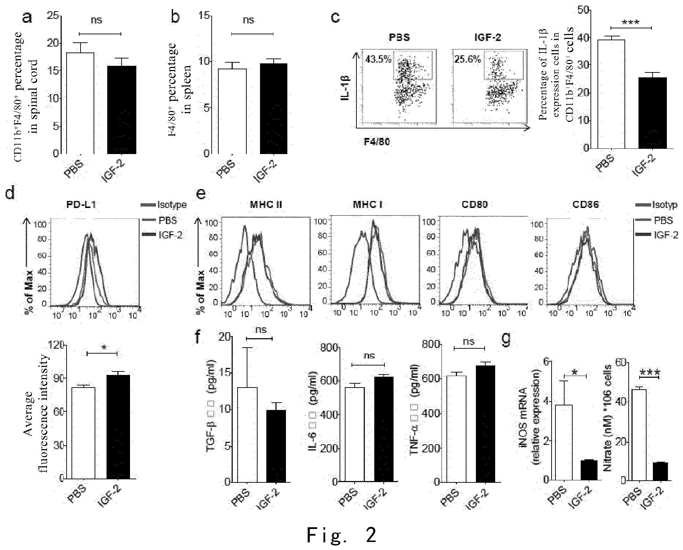

When flow cytometry instrument was used to analyze immune cells in EAE mice, it was also found that although the use of IGF₂₅₋₉₁ did not affect the percentage of macrophages in the spinal cord and spleen in EAE disease mice (Figures 2a - b), it could significantly affect the gene expression in macrophages, which was shown as significantly decreased expression of IL-1β and significantly increased expression of PD-L1 in macrophages (Figures 1i-j; Figures 2c-d). In conclusion, IGF₂₅₋₉₁ can be used in the treatment of EAE and can influence the ratio of different T cell subsets and the gene expression of macrophages in diseased mice.

### Example 2

### Macrophages treated with IGF₂₅₋₉₁ can promote the production of Treg and treat EAE

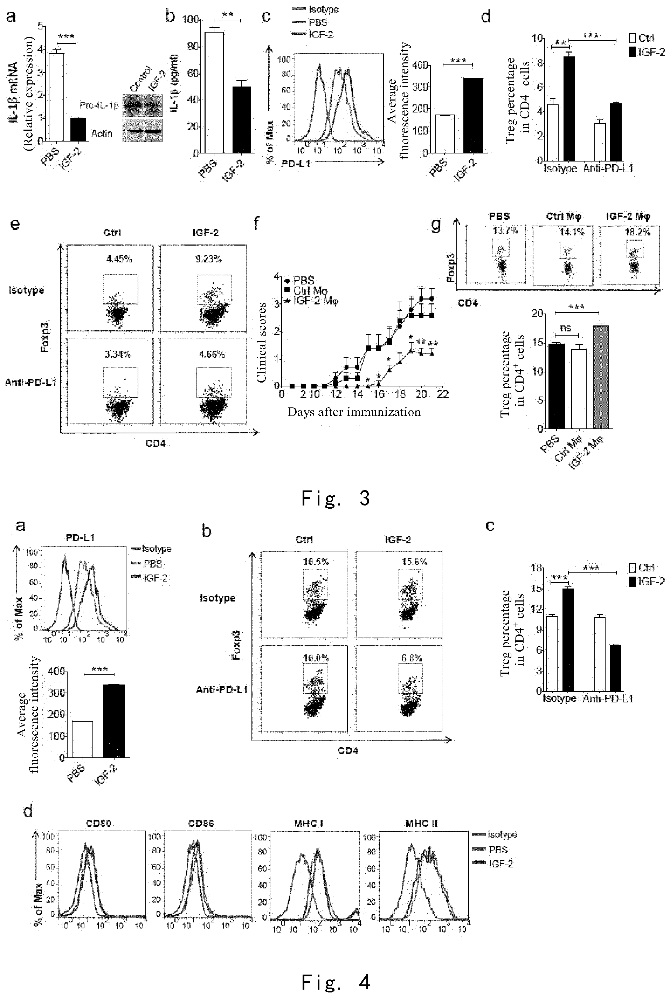

In order to further study the effect of IGF₂₅₋₉₁ on macrophages, two classical methods were used to prepare macrophages for research. One was inducing peritoneal macrophages with thioglycollate, and the other was differentiating macrophages using bone marrow cells stimulated by L929 cell culture supernatant. It was found that after receiving IGF₂₅₋₉₁ treatment during the differentiation of macrophages, there was no significant change in the expression of co-stimulating molecules (CD80, CD86, MHC I, MHC II), TNF-α, IL-6, and TGF-β in peritoneal macrophages or bone marrow derived macrophages (Figure 2e-f; Figure 4 d). However, after lipopolysaccharide stimulation, compared with control macrophages, the expression of IL-1β was significantly decreased, while the expression of PD-L1 was significantly increased in insulin-like growth factor-2 treated macrophages, which was quite consistent with the observed phenomenon *in vivo* (Figures 3a-c). In order to study the effect of macrophages treated with IGF₂₅₋₉₁ on Treg differentiation, macrophages and T cells were co-cultured, and anti-CD3, anti-CD28 and TGF-β required for Treg differentiation were added into the culture system. The results showed that, compared with normal macrophages, macrophages treated with IGF₂₅₋₉₁ (including bone marrow derived macrophages and peritoneal derived macrophages) could promote Treg production more effectively after LPS pre-stimulation. At the same time, PD-L1 played a crucial role in this process, because once a neutralizing antibody against PD-L1 was used, the Treg-promoting capacity of macrophages treated with IGF₂₅₋₉₁ was inhibited (Figures 3 d-e; Figures 4 a -c).

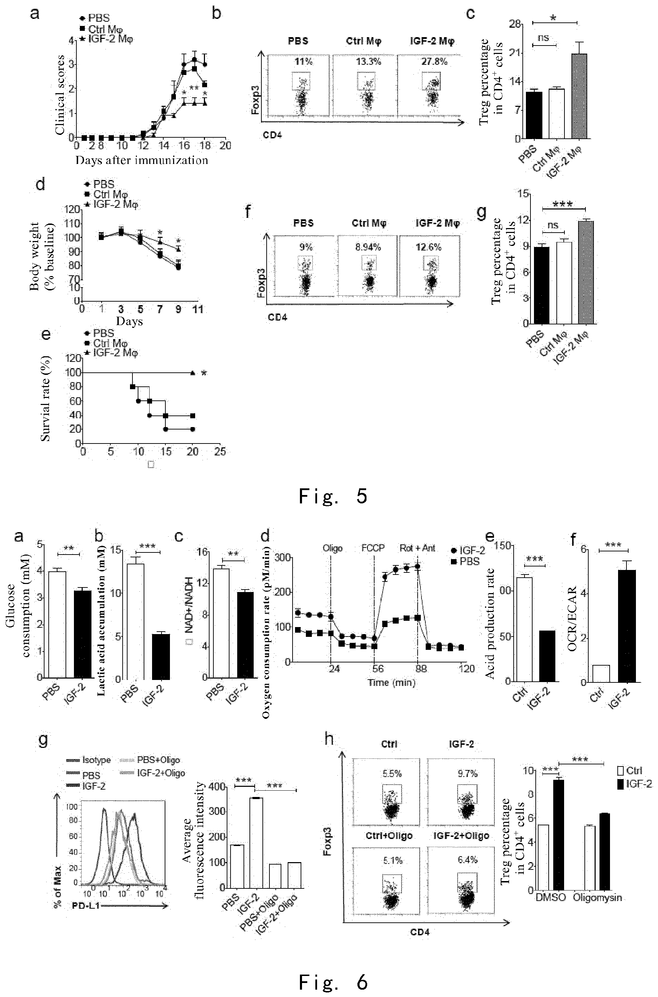

In order to further validate the immune regulation ability of macrophages treated with IGF₂₅₋₉₁, the treated macrophages (including bone marrow derived macrophages and peritoneal derived macrophages) were used in the treatment of EAE. The results showed that macrophages pretreated with IGF₂₅₋₉₁ could effectively alleviate the clinical scores of EAE, and promote the proportion of Treg in the spleen of EAE mice (Figures 3 f-g; Figures 5 a-c).

### Example 3

### IGF₂₅₋₉₁ affected gene expression of macrophages through elevating oxidative phosphorylation metabolic pathway

To investigate the regulatory mechanism of IGF₂₅₋₉₁ on macrophages, the properties of IGF₂₅₋₉₁ pre-treated macrophages and control macrophages were compared and it was found that both glucose consumption and lactate accumulation were decreased in IGF₂₅₋₉₁ pre-treated macrophages (Figures 6 a-b). NAD+/NADH ratio in macrophages was also reduced after IGF₂₅₋₉₁ treatment (Figure 6c), suggesting that IGF₂₅₋₉₁ could regulate the metabolic pathways of glycolysis and oxidative phosphorylation in macrophages. When tracking the oxygen consumption and acid production in macrophages, it was found that IGF₂₅₋₉₁ could effectively improve the efficiency of the oxygen consumption in macrophages, and at the same time reduce the acid production in macrophages in the background state (FIG. 6 d-f).This suggests that IGF₂₅₋₉₁ can tilt macrophage metabolism toward the oxidative phosphorylation pathway.

In order to confirm that the metabolic state of oxidative phosphorylation was the basis for the high expression of PD-L1 in macrophages and the promotion of Treg differentiation, oligomycin ( an inhibitor of oxidative phosphorylation process) was used. It was found that once the oxidative phosphorylation was blocked, the high expression of PD-L1 in macrophages and the ability to promote the differentiation of Treg caused by IGF₂₅₋₉₁ disappeared (FIG. 6 g-h). This suggests that the high expression of PD-L1 in macrophages and the ability to promote the differentiation of Treg induced by IGF₂₅₋₉₁ depend on the metabolic mode of oxidative phosphorylation.

### Example 4

### IGF₂₅₋₉₁ and IGF₂₅₋₉₁ pre-treated macrophages are effective in treating inflammatory bowel diseases (IBD).

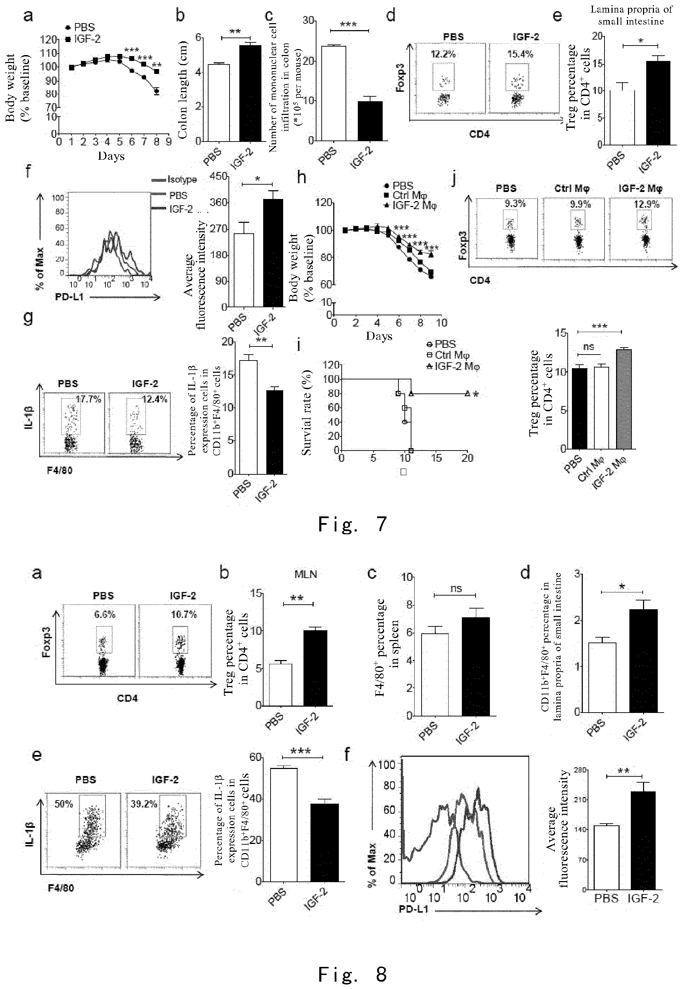

To further validate the therapeutic effects of IGF₂₅₋₉₁ on autoimmune diseases and the regulation of macrophages, IGF₂₅₋₉₁ was used to treat IBD, an autoimmune disease dominated by the innate immune system. After treatment with IGF₂₅₋₉₁, the weight loss of IBD mice was alleviated, colon injury and infiltration of monocytes in the colon were significantly reduced (Figures 7 a-c). Flow cytometry was used to analyze T cell populations and macrophage gene expression, and it was found that IGF₂₅₋₉₁ could increase Treg percentages in the colon and mesenteric lymph nodes (Figures 7 d-e; Figures 8 a-b). Meanwhile, the decreased expression of IL-1β and the increased expression of PD-L1 in macrophages were observed in the colon and spleen (Figures 7 f-g; Figures 8 c-f). Moreover, IGF₂₅₋₉₁ pre-treated macrophages could be administered directly in the treatment of IBD to alleviate body weight loss and improve the survival rate of IBD mice (Figures 5 d-e; Figures 7 h-i). IGF₂₅₋₉₁ pre-treated macrophages also increase Treg percentages in the spleen, peyer's patches and mesenteric lymph nodes (Figures 5 f-g; Figures 7 h-j).

### Example 5

### IGF₂₅₋₉₁ and macrophage scavenger are more effective in treating inflammatory diseases

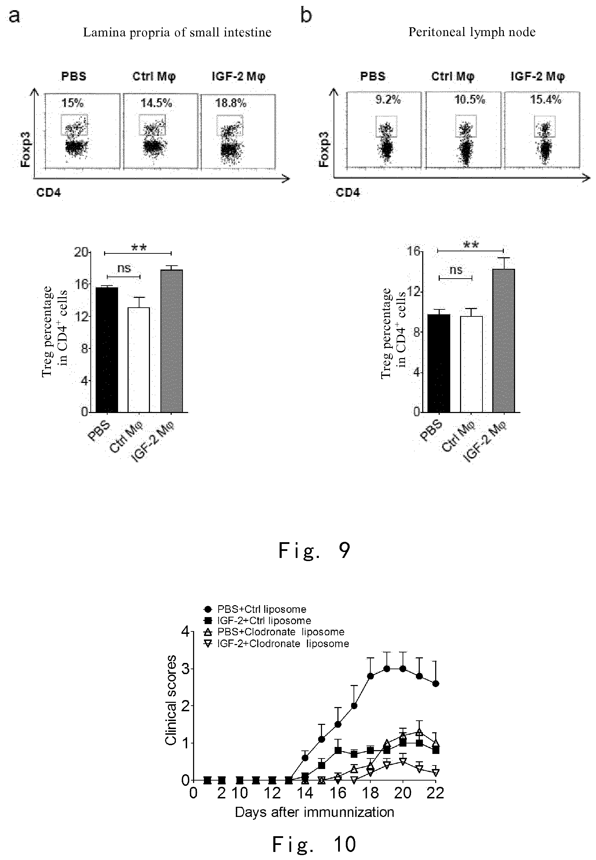

IGF₂₅₋₉₁ can significantly inhibit inflammatory diseases, such as the above-mentioned multiple sclerosis and inflammatory bowel diseases. Moreover, this effect is closely related to the IGF₂₅₋₉₁ regulation on macrophages which are reprogrammed into macrophages with immunosuppressive function, thus inhibiting over-strong inflammatory response. However, when inflammatory disease occurs, systemic and local inflammation is often accompanied by a large amount of proinflammatory macrophage infiltration, and the macrophage infiltration in inflammatory site often depends on an influx of peripheral monocytes and its evolution into proinflammatory macrophages. Therefore, we investigated whether a combination of clearance of resident proinflammatory macrophages and the reprogramming function of IGF₂₅₋₉₁ on macrophages can optimize the treatment of inflammatory disease. On the 9th, 11th, and 13th day of EAE induction, mice in different experimental groups received IGF₂₅₋₉₁, Clodronate liposome (for removing macrophage) or a combined injection of IGF₂₅₋₉₁ and Clodronate liposome. The progress of EAE in mice was observed. It was found that single injection of IGF₂₅₋₉₁ and Clodronate liposome could significantly inhibit the progress of EAE and had an effective therapeutic effect compared with the control group. More importantly, a combined injection of IGF₂₅₋₉₁ and Clodronate liposome had a more significant therapeutic effect on EAE compared with other groups (Figure 10). Therefore, the combination therapy of IGF₂₅₋₉₁ and macrophage scavenger has a more optimized effect in the treatment of inflammatory diseases by reprogramming cells into macrophages with immunosuppressive function.

### Example 6

### IGF₂₅₋₉₁ pre-treated macrophages can be used to treat obesity-induced insulin resistance.

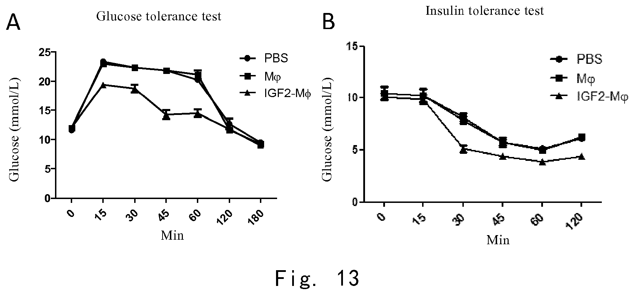

Mice were fed with high fat diet for 20 weeks and then injected with PBS, macrophages (5×10⁶) or IGF₂₅₋₉₁ pre-treated macrophages (5×10⁶). Two weeks after the treatment, glucose tolerance test and insulin tolerance test were conducted, and glucose changes in each group were detected. The results showed that IGF₂₅₋₉₁ pre-treated macrophages could significant improve the ability of glucose tolerance in the insulin resistance mice (Figures 13A and 13B). These results demonstrated that IGF₂₅₋₉₁ pre-treated macrophages were effective in treating insulin resistance and diabetes.

It should be understood that, after reading the above contents, the skilled person can make various modifications or amendments to the present invention which also fall into the scope defined by the claims.

## Claims

1. Macrophage treated with insulin-like growth factor-2 (IGF-2) for use in the treatment of an autoimmune disease.

2. The macrophage treated with IGF-2 for use of claim 1, wherein the autoimmune disease is selected from the group consisting of multiple sclerosis, inflammatory bowel disease, autoimmune encephalomyelitis, autoimmune hepatitis, systemic lupus erythematosus, rheumatoid arthritis, insulin resistance, diabetes, liver cirrhosis and a combination thereof.

3. The macrophage treated with IGF-2 for use of claim 2, wherein the insulin resistance or the diabetes is obesity-induced insulin resistance or obesity-induced diabetes.

4. The macrophage treated with IGF-2 for use of claim 1, wherein the IGF-2 includes full length IGF-2 and an active fragment thereof.

5. The macrophage treated with IGF-2 for use of claim 4, wherein the active fragment of IGF-2 is an active fragment having amino acids from position 25 to position 91 of IGF-2.

6. The macrophage treated with IGF-2 for use of claim 1, wherein the amino acid sequence of the IGF-2 is shown in SEQ ID NO: 1.

7. A pharmaceutical composition comprising (a) insulin-like growth factor-2 or an active fragment thereof, (b) macrophage scavenger, and (d) a pharmaceutically acceptable carrier; wherein the macrophage scavenger is selected from the group consisting of: CCR2 inhibitor, clodronate disodium liposome and a combination thereof.

8. The pharmaceutical composition of claim 7, wherein the active fragment of IGF-2 is an active fragment having amino acids from position 25 to position 91 of IGF-2.

9. A pharmaceutical composition for use in the treatment of an autoimmune disease, which comprises macrophage treated with insulin-like growth factor-2 and a pharmaceutically acceptable carrier.

10. An *in vitro* non-therapeutic method for (i) promoting PD-L1 expression and/or (ii) inhibiting IL-1β expression in macrophage, which comprises the following step (a) culturing macrophage in the presence of insulin-like growth factor-2, thereby (i) promoting PD-L1 expression in macrophage and/or (ii) inhibiting IL-1β expression in macrophage.

## Patentansprüche

1. Makrophage, der mit Insulin-like Growth Factor 2 (IGF-2) behandelt ist, zur Verwendung bei der Behandlung einer Autoimmunerkrankung.

2. Makrophage, der mit IGF-2 behandelt ist, zur Verwendung gemäß Anspruch 1, wobei die Autoimmunerkrankung aus der Gruppe ausgewählt ist, die aus multipler Sklerose, entzündlicher Darmerkrankung, Autoimmun-Enzephalomyelitis, Autoimmun-Hepatitis, systemischem Lupus erythematodes, rheumatoider Arthritis, Insulinresistenz, Diabetes, Leberzirrhose und einer Kombination davon besteht.

3. Makrophage, der mit IGF-2 behandelt ist, zur Verwendung gemäß Anspruch 2, wobei es sich bei der Insulinresistenz oder dem Diabetes um Adipositas-induzierte Insulinresistenz oder Adipositas-induzierten Diabetes handelt.

4. Makrophage, der mit IGF-2 behandelt ist, zur Verwendung gemäß Anspruch 1, wobei das IGF-2 ein IGF-2 der vollen Länge und ein aktives Fragment davon umfasst.

5. Makrophage, der mit IGF-2 behandelt ist, zur Verwendung gemäß Anspruch 4, wobei das aktive Fragment von IGF-2 ein aktives Fragment ist, das Aminosäuren von Position 25 bis Position 91 von IGF-2 aufweist.

6. Makrophage, der mit IGF-2 behandelt ist, zur Verwendung gemäß Anspruch 1, wobei die Aminosäuresequenz des IGF-2 in SEQ ID Nr. 1 gezeigt ist.

7. Pharmazeutische Zusammensetzung, umfassend (a) Insulin-like Growth Factor 2 oder ein aktives Fragment davon, (b) Makrophagen-Scavenger und (d) einen pharmazeutisch annehmbaren Träger;
wobei der Makrophagen-Scavenger aus der Gruppe ausgewählt ist, die aus folgenden besteht: CCR2-Inhibitor, Dinatriumclodronat-Liposom und einer Kombination davon.

8. Pharmazeutische Zusammensetzung gemäß Anspruch 7, wobei das aktive Fragment von IGF-2 ein aktives Fragment ist, das Aminosäuren von Position 25 bis Position 91 von IGF-2 aufweist.

9. Pharmazeutische Zusammensetzung zur Verwendung bei der Behandlung einer Autoimmunerkrankung, umfassend einen mit Insulin-like Growth Factor 2 behandelten Makrophagen und einen pharmazeutisch annehmbaren Träger.

10. Nichttherapeutisches In-vitro-Verfahren zur (i) Förderung der PD-L1-Expression und/oder (ii) Hemmung der IL-1β-Expression in Makrophagen, das den folgenden Schritt umfasst:
(a) Kultivieren von Makrophagen in Gegenwart von Insulin-like Growth Factor 2, wodurch (i) die PD-L1-Expression in Makrophagen gefördert wird und/oder (ii) die IL-1β-Expression in Makrophagen gehemmt wird.

## Revendications

1. Macrophage traité avec le facteur de croissance analogue à l'insuline 2 (IGF-2) pour une utilisation dans le traitement d'une maladie auto-immune.

2. Macrophage traité avec l'IGF-2 pour une utilisation selon la revendication 1, dans lequel la maladie auto-immune est choisie dans le groupe constitué de la sclérose en plaques, la maladie inflammatoire chronique de l'intestin, l'encéphalomyélite auto-immune, l'hépatite auto-immune, le lupus érythémateux disséminé, l'arthrite rhumatoïde, la résistance à l'insuline, le diabète, la cirrhose du foie et une combinaison de celles-ci.

3. Macrophage traité avec l'IGF-2 pour une utilisation selon la revendication 2, dans lequel la résistance à l'insuline ou le diabète est une résistance à l'insuline induite par l'obésité ou un diabète induit par l'obésité.

4. Macrophage traité avec l'IGF-2 pour une utilisation selon la revendication 1, dans lequel l'IGF-2 comporte l'IGF-2 complet et un fragment actif de celui-ci.

5. Macrophage traité avec l'IGF-2 pour une utilisation selon la revendication 4, dans lequel le fragment actif de l'IGF-2 est un fragment actif ayant des acides aminés de la position 25 à la position 91 de l'IGF-2.

6. Macrophage traité avec l'IGF-2 pour une utilisation selon la revendication 1, dans lequel la séquence d'acides aminés de l'IGF-2 est indiquée dans SEQ ID NO: 1.

7. Composition pharmaceutique comprenant (a) le facteur de croissance analogue à l'insuline 2 ou un fragment actif de celui-ci, (b) un agent de piégeage des macrophages, et (d) un support pharmaceutiquement acceptable ; dans laquelle l'agent de piégeage des macrophages est choisi dans le groupe constitué par l'inhibiteur de CCR2, le liposome disodique de clodronate et une combinaison de ceux-ci.

8. Composition pharmaceutique selon la revendication 7, dans laquelle le fragment actif de l'IGF-2 est un fragment actif ayant des acides aminés de la position 25 à la position 91 de l'IGF-2.

9. Composition pharmaceutique pour une utilisation dans le traitement d'une maladie auto-immune, qui comprend un macrophage traité avec le facteur de croissance analogue à l'insuline 2 et un support pharmaceutiquement acceptable.

10. Procédé non thérapeutique *in vitro* pour (i) favoriser l'expression de PD-L1 et/ou (ii) inhiber l'expression de IL-1β dans le macrophage, qui comprend l'étape suivante :
(a) la culture de macrophage en présence de facteur de croissance analogue à l'insuline 2, ce qui (i) favorise l'expression de PD-L1 dans le macrophage et/ou (ii) inhibe l'expression d'IL-1β dans le macrophage.
